# EUROPEAN PATENT APPLICATION

(11) **EP 2 280 081 A1**
(43) Date of publication of application: **02.02.2011**
(21) Application number: 09167015.8
(22) Date of filing: 31.07.2009
(51) Int. Cl.: C12Q 1/68

(54) **Method of normalized quantification of RNA**

(71) Applicant: Qiagen GmbH, 40724 Hilden (DE)
(72) Inventor: Löffert, Dirk, 40589 Düsseldorf (DE); Korfhage, Christian, 40764 Langenfeld (DE); Engel, Holger, 40724, Hilden (DE)
(74) Representative: Vossius & Partner

(57) **Abstract**

The present invention is related to normalized quantification of RNAs and to the normalization of quantities of RNAs in samples, e.g. mixtures of RNAs. The present invention relates to method for the normalization of the quantity of a RNA to be quantified in a sample to the total quantity of RNA in the sample; or to the total quantity of a specific class of RNA in the sample.

## Description

### Technical field of the invention

The present invention is in the field of Biology and Chemistry. In particular, the invention is in the field of Molecular Biology. More particular, the invention is in the field of quantification of nucleic acids and real-time PCR. Furthermore, the invention is related to normalized quantification of RNA and to the normalization of quantities of RNAs in samples, e.g. mixtures of RNAs.

### Background of the invention

The quantification (quantitation) of specific RNAs in mixtures of RNAs is of importance in a number of applications in molecular biology, such as gene expression analysis or during purification of specific nucleic acids from a mixture of nucleic acids. In quantification methods, the concentrations and/or the relative or absolute amounts of specific RNAs in samples are determined. In particular, for the analysis of gene expression, for example for measuring mRNA levels in biological samples, a reproducible and comparative method is desired. For example, it is not always possible to obtain biological samples with comparable volume, amount of RNA, cellular material or the like. Different samples can, for example, comprise RNA derived from different tissues, from different organisms or individuals, or cell culture samples that have been treated with different compounds.

In addition, sensitivity and selectivity of detection and quantification of RNAs in biological samples is of importance. For better comparison of the quantities of specific RNAs in two or more different (biological) samples or the comparison of the quantities of two or more different specific RNAs in a sample, a normalization of the quantities of the specific RNAs to the input RNAs or a specific class of input RNA has to be performed. Quantities of specific RNAs can, e.g., be normalized by relating these quantities to an internal standard of the sample or to the overall, *i*.*e*. total, amount of RNA or to the amounts of a specific class of RNA in the sample.

For conventional quantification of RNAs in (biological) samples, quantitative (real-time) PCR (qPCR) is widely used. For RNA, particularly mRNA, quantitative real-time reverse transcription PCR (RT-qPCR) is used in this field. Different approaches for the normalization of data obtained from quantitative PCR methods have been employed. Among them is the normalization of the quantities of specific mRNAs to the quantities of one or more mRNAs of different reference genes, e.g. housekeeping or maintenance genes, such as beta-actin, glyceraldehyde-3-phosphate dehydrogenase (GAPDH), hypoxanthine-guanine phosphoribosyl transferase (HPRT), or 28S or 18S ribosomal RNA. However, the expression levels of such normalizer genes have been shown to vary depending on experimental conditions, preparation and source (e.g. tissue or cell type) of the samples and therefore they are not reliably indicative for the input RNAs. It is therefore commonly required to test a range of different housekeeping genes in a laborious and error-prone procedure in order to identify those which do not charge between samples under investigation.

Other approaches, for example, rely on the normalization to the total content of DNA and/or RNA or the total content of e.g. ribosomal RNA (rRNA). As the content of ribosomal RNA in biological cells and samples is also subject to variations depending on a variety of factors, normalization to rRNA is also less preferred. Methods relying on the normalization to e.g. total RNA content, total RNA content or total content of genomic DNA are also limited, e.g. by variations in these contents or the quality of the RNA samples. Normalization to alien or artificial molecules, e.g. *in vitro* transcripts, that have been incorporated into a sample (e.g. a cell extract or a sample derived from a tissue) is also not in all cases an adequate procedure, since they do not represent the RNA (e.g. genomic DNA, RNA, mRNA) content in a cell.

Besides, for comparison of normalized data and reproducibility of the experimental procedures, thorough documentation of the applied experimental conditions is required. This is particularly relevant, when the quantities of the RNA of interest and the normalizer RNA are determined separately or using different methods.

Therefore, the technical problem underlying the present invention was to develop and to provide an improved, in particular a less laborious and error-prone, method for the normalization of quantities of RNAs or the cDNA generated from this RNA.

### Summary of the invention

The present invention provides (a) robust and improved method(s) for the normalization of the quantity of a (specific) RNA (*i*.*e*. a "target RNA") in a sample or in a plurality of samples to the total quantity of RNA in the sample(s); or the total quantity of a specific class of RNA in the sample(s). The present invention also relates to a kit for the normalization of the quantity of a (specific) RNA (*i*.*e*. a "target RNA") in a sample or in a plurality of samples to the total quantity of RNA in the sample(s); or the total quantity of a specific class of RNA in the sample(s).

In the context of the present invention, a specific class of RNA may be, *inter alia,* mRNA (messenger RNA), mtRNA (mitochondrial), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (doubled-stranded RNA), ribozyme, riboswitch, viral RNA, or the like or any other class or subclass ofRNA which is distinguishable from the bulk RNA in a sample.

The means and methods of the present invention comprise the use of nucleic acid probes. A nucleic acid probe according to the present invention is an oligonucleotide, nucleic acid or a fragment thereof, which is substantially complementary to a specific nucleic acid sequence.

In general, the present invention relates to a method for the normalization of the quantity of an RNA to be quantified in a sample to
(a) the total quantity of RNA in the sample; or to
(b) the total quantity of a specific class of RNA in the sample, comprising the steps of
   (i) providing a sample containing an RNA to be quantified;
   (ii) adding a first nucleic acid probe to the sample under conditions allowing for hybridization of at least a terminal region of said first nucleic acid probe to specific binding sites of RNA in the sample such that double-stranded nucleic acids are created, wherein said first nucleic acid probe comprises one or more primer binding sites and optionally a probe binding site;
   (iii) reverse transcribing the RNA in the sample, wherein the terminal region of the RNA serves as primer for cDNA synthesis and said first probe serves as template;
   (iv) quantifying the total amount of RNA in the sample or the total amount of the specific class of RNA in the sample optionally using a second probe substantially complementary to a region of the DNA transcribed from said RNA;
   (v) quantifying the RNA to be quantified optionally using a third probe which is substantially complementary to a defined region of the RNA to be quantified; and
   (vi) normalizing the quantity of the RNA to be quantified by determining the ratio of the quantity of the RNA to be quantified to the total quantity of RNA in the sample or the total quantity of the specific class of RNA in the sample.

The present invention also relates to a kit for performing any of the above described methods, wherein the kit comprises: a first nucleic acid probe substantially complementary to defined regions of RNA or a specific class of RNA in the sample, wherein the first nucleic acid probe comprises one or more primer binding sites and a probe binding site; and optionally one or more second nucleic acid probes substantially complementary to said probe binding site on said first nucleic acid probe.

### Detailed description of the invention

The present invention provides a method for the normalization of the quantity of an RNA to be quantified in a sample to
(a) the total quantity of RNA in the sample; or to
(b) the total quantity of a specific class of RNA in the sample,
comprising the steps of
(i) providing a sample containing an RNA to be quantified;
(ii) adding a first nucleic acid probe to the sample under conditions allowing for hybridization of at least a terminal region of said first nucleic acid probe to specific binding sites of RNA in the sample such that double-stranded nucleic acids are created, wherein said first nucleic acid probe comprises one or more primer binding sites and optionally a probe binding site;
(iii) reverse transcribing the RNA in the sample, wherein the terminal region of the RNA serves as primer for cDNA synthesis and said first probe serves as template;
(iv) quantifying the total amount of RNA in the sample or the total amount of the specific class of RNA in the sample optionally using a second probe substantially complementary to a region of the DNA transcribed from said RNA;
(v) quantifying the RNA to be quantified optionally using a third probe which is substantially complementary to a defined region of the RNA to be quantified; and
normalizing the quantity of the RNA to be quantified by determining the ratio of the quantity of the RNA to be quantified to the total quantity of RNA in the sample or the total quantity of the specific class of RNA in the sample.

The "total quantity of RNA" or the "total quantity of a specific class of RNA" refer to the reference quantities (reference RNA) for normalization of the quantity of the RNA to be quantified.

A sample in the context of the present invention contains nucleic acid molecules comprising the RNA to be quantified. The RNA can be embedded in cells or organisms but can also be present in cell free systems. A sample may be a fluid, a lysate, solid matrix anything else that contain RNA molecules. The sample may be of any origin, e.g. viral, bacterial, archae-bacterial, fungal, ribosomal, eukaryotic or prokaryotic. It may be a sample derived from any biological source and any organism, tissue, cell or sub-cellular compartment. It may e.g. be a sample from a plant, a fungus, an animal, and particularly a human sample. Also samples comprising artificial RNA may be employed in the context of the present invention.

In particular embodiments the RNA to be quantified is RNA selected from the group consisting of mRNA, rRNA, tRNA, nRNA, siRNA, snRNA, snoRNA, scaRNA, microRNA, dsRNA, ribozyme, riboswitch and viral RNA and wherein the specific class of RNA is selected from the group consisting of mRNA, rRNA, tRNA, nRNA, siRNA, snRNA, snoRNA, scaRNA, microRNA, dsRNA, ribozyme, riboswitch and viral RNA.

More in particular, the RNA to be quantified is mRNA and the specific class of RNA is mRNA.

In general, any RNA having a free 3'-OH end (and thus can be elongated) may be quantified in the context of the present invention.

The RNA to be quantified and the reference RNA may be of any origin, e.g. viral, bacterial, archae-bacterial, fungal, ribosomal, eukaryotic or prokaryotic. It may be RNA from any biological sample and any organism, tissue, cell or sub-cellular compartment. It may e.g. be RNA from a plant, a fungus, an animal, and particularly human RNA. The RNA may be pretreated before quantification, e.g. by isolation, purification or modification. Also artificial RNA may be quantified.

mRNA may be poly-adenylated messenger RNA as naturally occurring from different species. The RNA can also be any RNA sample, with our without poly-A tail, wherein a poly-nucleotide tail, e.g. a poly-A tail, is added in vitro before quantification by a suitable method, e.g. an enzyme, preferably a poly-A-polymerase.

Particularly, the specific binding site is specific for mRNA or is a poly-A sequence or a poly-A tail. The poly-A tail can be as naturally occurring in the sample RNA. In case the RNA does not have a poly-A tail, experimental procedures may in one embodiment be employed to add a suitable homopolymeric tail to the 3' end of the RNA. Such tail can be composed of of A, C ,G, or U bases or suitable base analogues. Addition of such tail can be performed chemically or enzymatically. Enzymes for this purpose may for example be selected from the group of poly-A polymerase, terminal transferase, ligase, or other suitable enzyme catalyzing addition or linkage of nucleotides to the 3' end of a nucleic acid.

The lengths of the poly-nucleotide-tail, e.g. the poly-A tail, as either naturally occurring or added in vitro, may vary in length from 1 nucleotide to several thousand of nucleotides, preferably from 1 nucleotide to several hundred nucleotides, most preferably from 5 to 100 nucleotides.

In one embodiment of the invention, RNase is added to the sample after the reverse transcription step under conditions allowing for digestion of RNA.

In particular, the RNase is selected from the group consisting of RNase A, RNase H, RNase I, RNase T and RNase activity of Reverse Transcriptase. Chemical and physical methods are also suitable to degrade RNA, e.g. a suitable pH change, elevated temperatures, cations or anions or a combination of one or more of such parameters. However, such methods are known to a skilled person.

In particular embodiments of the invention, said first nucleic acid probe is a DNA in which dT nucleotides are replaced by dU nucleotides and wherein a step of incubating the sample with uracil DNA glycosylase under conditions allowing for hydrolysis of the N-glycosylic bond between the uracil and sugar, is performed at least once before quantifying the total amount of RNA or the total amount of a specific class of RNA.

Additional primers can be employed in such a reverse transciption reaction, e.g. primers specific for certain RNA sequences, oligo dT primers, random primers of different length or selected degenerate primers, i.e. preferably primers not hybridizing significantly to the first nucleic acid probe, but allowing synthesis of cDNA from the RNA sample.

In some embodiments the 3' end of said first nucleic acid probe may be blocked. Different methodologies are known to block the 3' end of a nucleic acid, comprising incorporation of didesoxy-nucleotides, inverse base, a spacer, dye, quencher-moeity like e.g. Black Hole Quencher, Dabcyl, Minor-Groove-Binder, modified bases like e.g.. Super-Bases or halogenated bases, or base-analogues, abasic sites, blocking 3'-OH group (e.g. replacing it by phosphate group). Further, all other possible modifications of sugar-backbone, the bases or additional side chains, that inhibit the catalytic activity of suitable enzymes like reverse transcriptases or other enzymes suitable to perform the reaction in the scope of this invention may be used for blocking. Modifications may be performed enzymatically or chemically. Particularly, the quantifying steps comprise a method selected from the group consisting of gel electrophoresis, capillary electrophoresis, labelling reactions with subsequent detection measures and quantitative real-time PCR. Furthermore, quantification may also be based on hybridization of probes to cDNA and direct or indirect (e.g. in an amplification reaction such as real-time quantitative PCR) detection of the probes hybridized to the cDNA. In other words: The generated cDNA may be detected for quantification by hybridization of one or more probes to the cDNA complementary to the first nucleic acid probe. Alternatively, one or more elements can be introduced into the cDNA complementary to the first nucleic acid probe useful as priming site for subsequent detection reactions, e.g. a promoter site for a RNA polymerase, priming site for rolling circle amplification, one or more hybridization sites for probes for isothermal amplification, modified nucleotides carrying a label (e.g. fluorescent dye, radioactive isotope) or a reactive group allowing coupling to a secondary detection reagent (e.g. enzyme, haptene).

Preferably, quantification is PCR-based, more preferably quantification comprises quantitative real-time PCR.

Nucleic-acid amplification may alternatively be accomplished by any of the other various nucleic-acid amplification methods known in the art, including but not limited to ligase chain reaction (LCR), transcription-based amplification system (TAS), nucleic acid sequence based amplification (NASBA), rolling circle amplification (RCA), transcription-mediated amplification (TMA), self-sustaining sequence replication (3SR) and Qβ amplification

More preferably, the quantification comprises quantitative real-time reverse transcription PCR. In preferred embodiments of the invention, the quantification step(s) comprise(s) (i) the reverse transcription of RNA (e.g. mRNA) into DNA (e.g. cDNA) using a RNA-dependent DNA polymerase (*i*.*e*. a reverse transcriptase), (ii) the amplification of the DNA produced by reverse transcription using PCR, and (iii) the detection and quantification of the amplification products in real time.

The enzyme harving Reverse Transcriptase activity in the context of this invention is selected from the group comprising enzymes of different origin, including viral, bacteria, archae-bacteria and eukaryotic origin, especially thermostable organisms. This includes enzymes from introns, retrotransposons or Retroviruses. An enzyme with reverse transcriptase activity in the scope of the invention is an enzyme which has the ability to add to the 3' end of a desoxyribonuleic acid or a ribonucleic acid, hybridized to a complementary template desoxyribonuleic acid or ribonucleic acid or vice versa, one or more desoxyribonucleotides at suitable reaction conditions (e.g. buffer conditions), complementary to said template desoxyribonuleic acid or ribonucleic acid. This includes enzyme, that intrinsically harbour this ability, but also enzymes that were evolved or mutated in their gene sequence to gain such function or that gain such function by adjusting buffer or other reaction parameters.

Preferably, the enzyme having Reverse Transcriptase activity in the context of this invention is selected from the group comprising HIV reverse Transcriptase, M-MLV reverse Transcriptase, EAIV reverse Transcriptase, AMV reverse Transcriptase, *Thermus thermophilus* DNA Polymerase I, M-MLV RNAse H, Superscript, Superscript II, Superscript III, Monstersript (Epicentre), Omniscript, Sensiscript Reverse Transcriptase (Qiagen), ThermoScript und Thenno-X (both Invitrogen). This includes enzyme, that intrinsically harbour this ability, but also enzymes that were evolved or mutated in their gene sequence to gain such function or that gain such function by adjusting buffer or other reaction parameters. The enzyme may also have increased fidelity like e.g. AccuScript reverse Transcriptase (Stratagene). It is also obvious to those skilled in the art that one or more suitable enzyme with reverse transcriptase activity can be mixed to gain optimized conditions or novel features. This may include, amongst others, mixtures of e.g. a mesophilic and a thermophilic enzyme, or a mixture of an enzyme having RNase H activity and an enzyme being RNase H negative, or an enzyme with increased fidelity and an thermophilic enzyme. It is obvious to those skilled in the art that numerous other combinations are possible. The enzyme having reverse transcriptase activity is at least an enzyme allowing cDNA synthesis using the poly-nucleotide tail as primer and the adapter nucleic acid as template. Different enzymes are possible dependent on the type of nucleic acids used in the poly-A-Tail or the adapter nucleic acid. It may originate from mesophilic or thermophilic organisms, providing activity in a temperature range from 5°C to 100°C, more preferably from 10°C-80°C, most preferably from 15°C to 75°C.

In further preferred embodiments reverse transcribing and quantifying are performed in the same reaction container.

In particular embodiments the reverse transcriptase is a polymerase also used for amplification during the quantification steps.

Selective primers or random primers may be used in quantitative real-time PCR to quantify the cDNA of the first nucleic acid probe. Preferably, selective primers are used in real-time PCR. In a more preferred embodiment, selective primers allowing amplification of the cDNA generated from the first nucleic probe, optionally an additional oligonucleotide probe are used. Optionally, one or more additional selective primer sets are present in the reaction, optionally an additional oligonucleotide probe, allowing co-amplification (multiplex amplification) of one more target nucleic acid species to be quantified.

Preferably, the quantity of the RNA or cDNA to be quantified and the total quantity of RNA or cDNA or the total quantity of the specific class of RNA or cDNA are determined at the same time.

In some embodiments of the invention, the polymerase used for PCR, particularly quantitative real-time PCR, is a polymerase from a mesophilic or thermophile organism or a thermostable polymerase or is selected from the group consisting of *Thermus thermophilus* (Tth) DNA polymerase, *Thermus acquaticus* (Taq) DNA polymerase, *Thermotoga maritima* (Tma) DNA polymerase, *Thermococcus litoralis* (Tli) DNA polymerase, *Pyrococcus furiosus* (Pfu) DNA polymerase, *Pyrococcus woesei* (Pwo) DNA polymerase, *Pyrococcus kodakaraensis* KOD DNA polymerase, *Thermus filiformis* (Tfi) DNA polymerase, *Sulfolobus solfataricus* Dpo4 DNA polymerase, *Thermus pacificus* (Tpac) DNA poylmerase, *Thermus eggertsonii* (Teg) DNA poylmerase and *Thermus flavus* (Tfl) DNA polymerase.

In preferred embodiments of the invention the second and optional third nucleic acid probe are labelled with one or more fluorescent dye(s) and wherein the quantifying steps comprise detecting fluorescence signals in the sample.

More preferably, the second and optional third nucleic acid probes are fluorescently labelled probes selected from the group consisting of hybridization probe, hydrolysis probe and hairpin probe.

Particularly, the fluorescently labelled probes are labelled with a dye selected from the group consisting of Pacific Blue, FAM, VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Mega Stokes Dyes, Alexa Fluor Series, Bodipy Series of dyes, and PET or other compatible fluorescent dyes having comparable excitation and/or emission properties or other dyes detectable at different wavelengths as the dyes mentioned above.

In particular, the hybridization probe is a LightCycler probe or the hydrolysis probe is a TaqMan probe. In other embodiments the hairpin probe is selected from the group consisting of molecular beacon, Scorpion primer. Sunrise primer, LUX primer and Amplifluor primer, DNAzyme, Mnazyme and other compatible probe technologies.

Preferably, the means and methods according to the present invention are used for the normalization of gene expression levels.

In some embodiments of the present invention additionally a pre-quantified nucleic acid is added to the sample and the quantity of said pre-quantified nucleic acid is determined in the quantifying steps. Preferably, the pre-quantified nucleic acid is a mRNA.

Preferably, the quantities of two or more nucleic acids to be quantified are normalized simultaneously, i.e. at the same time.

The present invention also relates to a kit for performing any of the above described methods, wherein the kit comprises: a first nucleic acid probe ("adapter probe", "adapter oligonucleotide" or "adapter nucleic acid") substantially complementary to defined regions of RNA or a specific class of RNA in the sample, wherein the first nucleic acid probe comprises one or more primer binding sites and a probe binding site; and optionally a second nucleic acid probe substantially complementary to said probe binding site on said first nucleic acid probe.

Thus, the present invention relates to a kit for the normalized quantification of RNA in a sample, wherein the kit comprises: a first nucleic acid probe ("adapter probe", "adapter oligonucleotide" or "adapter nucleic acid") substantially complementary to defined regions of RNA or a specific class of RNA in the sample, wherein the first nucleic acid probe comprises one or more primer binding sites and a probe binding site; and a optionally second nucleic acid probe substantially complementary to said probe binding site on said first nucleic acid probe.

In a preferred embodiment of the kit, the specific class of nucleic acid is mRNA and the specific binding site is poly-A. Preferably, in this embodiment the first nucleic acid probe ("adapter probe") comprises at its 5' end a poly-U or poly-T sequence with between about 1 and 150, more preferably between about 2 and 100, most preferably between about 4 and 80 U and/or T nucleotides.

The adapter probe may comprise one or more primer and/or probe binding sequences so that when the adapter probe serves as a template in the elongation of the reference RNA, the newly synthesized strand (complementary to the adapter probe) comprises one or more primer or probe binding sites. Primer and/or probe binding sites are sequences that otherwise do not occur in the nucleic acid to be quantified or in the RNA or class of RNA that serves as a reference for normalization. These primer and/or probe binding sites have substantially complementary sequences to primers and/or second nucleic acid probes used for amplification or quantification. Preferably, the adaptor probe comprises at least one probe binding site and two primer binding sites (forward and reverse primer).

The kit may in some particular embodiments additionally comprise one or more second nucleic acid probes and/or primers as outlined above. The kit may also comprise dNTPs and/or buffer substances or salts, e.g. Mg²⁺.

In preferred embodiments of the invention, the kit additionally comprises a polymerase and a set of primers and/or a reverse transcriptase. In some embodiments the kit additionally comprises a reverse transcriptase.

In some embodiments, one ore more of the components are premixed in the same reaction container.

In some embodiments the kit may additionally comprise one or more pre-quantified calibrator RNA(s), a set of primers for the amplification of said calibrator RNA(s) and a first nucleic acid probe substantially complementary to a sequence on said pre-quantified RNA(s).

In some embodiments, one ore more of the components are premixed in the same reaction container.

As indicated herein above, for the analysis of gene expression, the quantification of mRNA in samples can be performed using quantitative real-time reverse transcription PCR (RT-qPCR). RT-qPCR methods employ a combination of three steps: (i) the reverse transcription of the mRNA into cDNA using a RNA-dependent DNA polymerase (*i*.*e*. a reverse transcriptase), (ii) the amplification of cDNA using PCR, and (iii) the detection and quantification of the amplification products in real time. For reverse transcription and PCR-based amplification, dNTPs ("nucleotide mixture") need to be present in the reaction buffer. A nucleotide mixture according to the present invention is a mixture of dNTPs, *i.e.* a mixture of dATP, dCTP, dGTP and dTTP/dUTP suitable for the use in PCR. The mixture may optionally contain modified nucleotides or labelled nucleotides or nucleotide analoga. For particular embodiments of the present invention the relative amounts of these dNTPs may be adapted according to the particular nucleotide content of the template nucleic acids. The RT-qPCR steps can either be performed in a single-stage process or in a two-stage process. In the first case, reverse transcription and PCR-based amplification are performed in the same reaction container, e.g. by utilizing a DNA polymerase which has intrinsic reverse transcription functionality, like *Thermus thermophilus* (Tth) polymerase. In a two-stage setup the steps of reverse transcribing the RNA and amplifying the DNA are performed separately, e.g. in different reaction containers. The steps of the methods according to the present invention may be conducted in suitable reaction buffers, e.g. comprising salts such as magnesium ions. As already stated, the different steps may or may not be conducted in the same buffers and reaction containers. In contrast to RT-qPCR, in qPCR no reverse transcription is performed, therefore it is a quantification method for DNA rather than for RNA.

Suitable buffer solutions providing one or more buffer substances, a suitable pH, salts and cofactors like Mg²⁺ as well as suitable reaction conditions, e.g. temperatures, for amplification reactions are known to a skilled person.

The reverse transcription of (m)RNA in RT-qPCR and the amplification of (c)DNA in qPCR and RT-qPCR need to be primed by oligonucleotides ("primers"). In the case of mRNA quantification with RT-qPCR, mRNA specific oligonucleotides can be used, e.g. oligo-dT primers that hybridize to the poly-A-tail of mRNA. However, also random primers of varying lengths can be utilized or selective degenerate primer may be introduced.

Moreover, standard quantitative real-time PCR protocols and kits can be adapted or amended for the means and methods according to the present invention.

As mentioned above, real-time PCR (also designated herein as quantitative PCR or quantitative real-time PCR (qPCR)) is a method to simultaneously amplify and quantify nucleic acids using a polymerase chain reaction (PCR). Quantitative real-time reverse transcription PCR (RT-qPCR) is a quantitative real-time PCR method further comprising a reverse transcription of RNA into DNA, e.g. mRNA into cDNA. In qPCR and RT-qPCR methods, the amplified nucleic acid is quantified as it accumulates. Typically, fluorescent dyes that intercalate with double-stranded DNA (e.g. ethidiumbromide or SYBR® Green I) or modified nucleic acid probes ("reporter probes") that fluoresce when hybridized with a complementary nucleic acid (e.g. the accumulating DNA) are used for quantification in qPCR based methods. Particularly, fluorogenic primers, hybridization probes (e.g. LightCycler probes (Roche)), hydrolysis probes (e.g. TaqMan probes (Roche)), or hairpin probes, such as molecular beacons, Scorpion primers (DxS), Sunrise primers (Oncor), LUX primers (Invitrogen), Amplifluor primers (Intergen) or the like can be used as reporter probes. In accordance with the present invention, fluorogenic primers or probes may for example be primers or probes to which fluorescence dyes have been attached, e.g. covalently attached. Such fluorescence dyes may for example be FAM (5-or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor, Pacific Blue, Mega Stokes Dyes, Bodipy Series of dyes, and PET or other compatible fluorescent dyes showing comparable excitation and/or emission properties or other dyes detectable at different wavelengths as the dyes mentioned before and the like. Particular reporter probes may additionally comprise fluorescence quenchers.

The first nucleic acid probe, referred to herein also as "adapter nucleic acid", in the context of the present invention is a nucleic acid oligonucleotide or polynucleotide (RNA, DNA or an analogue thereof) having from about 10 to several 1000 nucleotides, more preferably from about 20 to 500 nucleotides and most preferably from about 30 to 150 nucleotides. The adapter nucleic comprises a first part (the "hybridizing element") with a sequence being substantially complementary to specific binding sites (specific sequences) of RNA, e.g. the poly-A tail of RNA, and a second part (the "adapter sequence"). The adapter sequence comprises one or more primer binding sites and optionally a probe binding site. The adapter sequence is preferably selected to have sufficient lengths to allow for specific detection of the cDNA in a detection method, generated using this sequence as template. One preferred detection method is PCR. In this case, the adapter sequence has a sufficient length allowing for at least one PCR primer to hybridize, more preferably two PCR primers and optionally one or more sequence specific probes. The 3' OH group of this adapter nucleic acid can be blocked to prevent extension or can be free, to allow cDNA synthesis. Appropriate methods to block the 3' OH are standard methods and known in the art. Suitable methods may be 3' phosphate group, inverse base, aliphatic linker, amino modifier, or others. The first part ("hybridizing element") of the adapter nucleic acid is preferably 3' in the sequence relative to the second part (adapter sequence). In case the specific binding site is a poly-A tail, the first part of the adapter nucleic acid (the "hybridizing element") may comprise U and/or T bases, particularly may be a poly-U or poly-T sequence. In a preferred embodiment, the lengths of the hybridizing element is between 1 and 150, more preferably between 2 and 100, most preferably between 4 and 80 nucleotides. Useful lengths may be limited by available production or synthesis technologies. The second part of the adapter nucleic acid may have a length of from about 5 to several 1000 nucleotides, more preferably from about 10 to 500 nucleotides and most preferably from about 20 to 140 nucleotides. The specific binding site may be a sequence specific for a particular class of RNA, e.g. a poly-A tail in the case of mRNA.

The second, third and potential further nucleic acid probes are oligonucleotides or polynucleotides having a length of from about 10 to 100 nucleotides, more preferably from about 12 to 50 nucleotides and most preferably from about 14 to 30 nucleotides having a sequence substantially taken from the sequence of the first nucleic acid probe or the complementary nucleic acid sequence (e.g. cDNA sequence) of a sequence of the first nucleic acid probe .These probes may be modified, particularly fluorescently labelled. They may be used in quantitative real-time PCR and may be reporter probes as outlined above.

A skilled person knows how to choose the length and sequence of the probes and primers depending on the reaction temperature of the elongation reaction, on the particular enzyme(s) used (e.g. thermostable polymerase, reverse transcriptase) and on the sequence of the respective binding partners.

The present invention also relates to the use of the methods of the invention or the kit of the invention for the normalization of the quantity of a specific nucleic acid to the quantity of a reference nucleic acid.

Furthermore, the present invention also relates to the use of the methods of the invention or the kit of the invention gene expression analysis.

### Examples

### Example 1: Realization of the technical concept of the method for normalized quantification of RNA using an adapter nucleic acid and detection of cDNA in Real-time PCR.

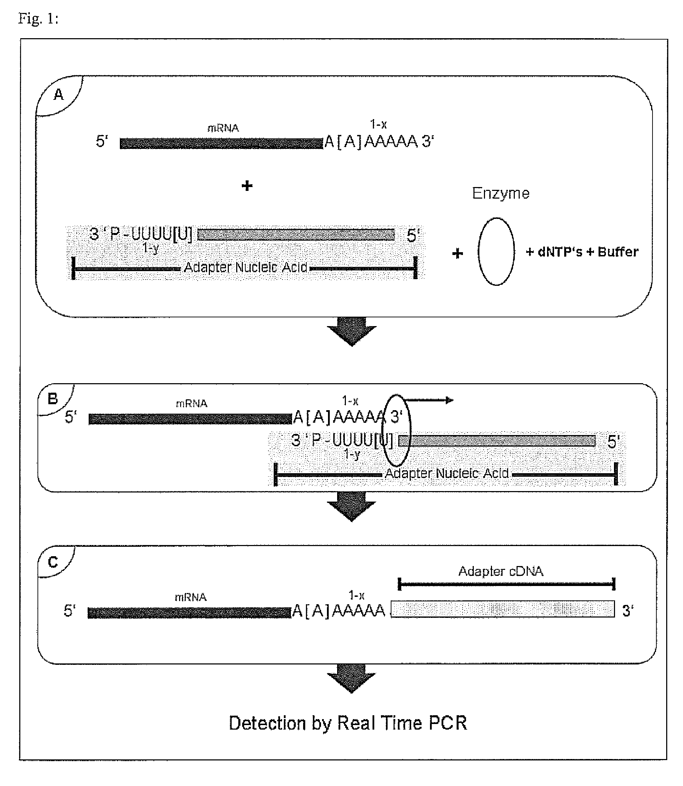

In this experiment the feasibility of the technical concept shown in Fig 1 shall be demonstrated. The reactions have been composed as shown in table 1 and carried out with the protocol shown in table 2. The reaction volume depends on the amount of RNA which should be detected (see table 1). For this purpose the reagents of table 3 were used. Dilutions of total RNA (isolated from Hela cells using an RNeasy Kit (QIAGEN) serve as template in cDNA synthesis. As a control for background signal, a negative control reaction cDNA synthesis reaction with 1 µg total Hela RNA and all components except the adapter Nucleic Acid.

| **Table 1: Setup for cDNA synthesis using an adapter nucleic acid** | |
|---|---|
| Component | Final concentration |
| Buffer RT,10x | 1x |
| RNAse Inhibitor, 40Units/µl | 2 Units/µl |
| 10mM dNTP Mix | 0,5mM each dNTP |
| Omniscript Reverse Transkriptase, 4Units/µl | 0,2 Units/µl |
| Adapter Nucleic Acid | 0,2µM |
| RNAse free water | variabel |
| RNA Sample concentration | Total RNA concentraton: 0,2-2,0 ng/µl) |
| Reaction volume | 500 µl |

| **Table 2: Protocol for cDNA synthesis** | | |
|---|---|---|
| Reverse transcription | 37°C for 45 minutes | |
| Inactivation of Reverse Transcription Enzyme | 95°C for 5 minutes | |
| | 4°C Hold | |

| **Table 3: Components and ordering information of materials for cDNA synthesis** | |
|---|---|
| Buffer RT, 10x | Components of QIAGEN - Omniscript RT Kit Material No. 205111 |
| Omniscript Reverse Transkriptase, 4Units/µl | |
| RNAse free water | |
| RNAse Inhibitor, 40Units/µl | Promega Material No. N251 B |
| 10mM dNTP Mix | Amersham Material No. 27-2074-60 |
| Adapter Nucleic Acid | RNA Oligonucleotide, 3' blocked by Amino-modifier: |
| | GGT GAG TGA TTG GAG GGT TGA GCA CAT CAG AGC CCT GCG ATG AGT CTG TCG TCG TCT CGT TCC AUU UUU UUU UUU UUU UU-3' NH2 |

After reverse trancription, the generated cDNA was then used as template in SYBR Green based real-time PCR, in order to analyse if a correlation between the observed Ct-Value in real-time PCR and the amount of RNA used in cDNA synthesis can be observed.

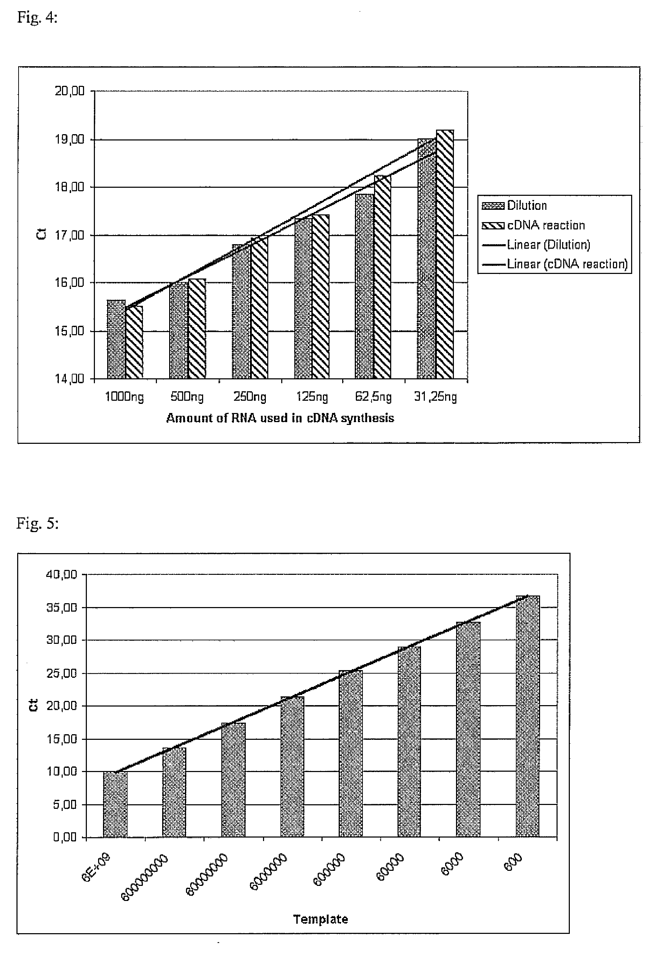

To this end, after inactivation of the enzyme (see example 1 table 2: 5 minutes at 95°C) 5µl of the cDNA synthesis reaction was applied as template to each reaction in the following real-time PCR. The detection in Realtime PCR was carried out with SYBR Green chemistry. Table 9 shows the setup for a SYBR Green Realtime PCR reaction. The PCR reaction was prepared with the components from table 8. Afterwards the cycling shown in table 10 was performed. Realtime PCR was made on a Applied Biosystems 7500 Realtime PCR System. Subsequently the rundata were analyzed with the appropriate software. The results are shown in table 11 and in figure 4.

| Table 8: Components and material numbers for SYBR Green Realtime PCR setup | |
|---|---|
| QuantiTect SYBR Green PCR Mastermix, 2x | Components of QIAGEN - QuantiTect SYBR Green PCR Kit Material No. 204143 |
| RNAse free water | |
| HUM-Uni | AAC GAG ACG ACG ACA GAC |
| HBSR 1 | GGT GAG TGA TTG GAG GGT TG |

| Table 9: Setup for RealTime PCR with SYBR Green | |
|---|---|
| Components | Final concentration |
| QuantiTect SYBR Green PCR Mastermix, 2x | 1x |
| HUM-Uni | 0,4µM |
| HBSR 1 | 0,4µM |
| RNAse free water | Top up to 20µl per reaction |
| Final reaction volume | 25µl |

| Table 10: Protocol for RealTime PCR with SYBR Green | | |
|---|---|---|
| PCR initial reactivation | 95°C for 15 minutes | |
| Denaturing | 95°C for 15 seconds | 40x |
| Annealing | 55°C for 30 seconds | |
| Extension (Data acquisition) | 70°C for 30 seconds | |
| Dissociation stage | | |

| Table 11: Results of SYBR Green Realtime PCR from a 500µl cDNA synthesis reaction. Comparison between dilution and Template from cDNA synthesis. | | | | | | | |
|---|---|---|---|---|---|---|---|
| Detector | Amount of RNA used in cDNA synthesis | Template in RealTime PCR | Ct | CV in % | Template in RealTime PCR | Ct | CV in % |
| SYBR | 1µg Hela RNA | 5µl of cDNA synthesis | 15,64 | 1,533 | 5µl of cDNA synthesis | 15,51 | 0,361 |
| SYBR | 500ng Hela RNA | 5µl of cDNA synthesis | 16,00 | 0,439 | 5µl of 1:2 dilution from Template above equates to 500ng Hela RNA in cDNA synthesis | 16,07 | 0,000 |
| SYBR | 250ng Hela RNA | 5µl of cDNA synthesis | 16,80 | 0,419 | 5µl of 1:2 dilution from Template above equates to 250ng Hela RNA in cDNA synthesis | 16,94 | 0,422 |
| SYBR | 125ng Hela RNA | 5µl of cDNA synthesis | 17,34 | 1,167 | 5µl of 1:2 dilution from Template above equates to 125ng Hela RNA in cDNA synthesis | 17,42 | 0,573 |
| SYBR | 62,5ng Hela RNA | 5µl of cDNA synthesis | 17,85 | 1,573 | 5µl of 1:2 dilution from Template above equates to 62,5ng Hela RNA in cDNA synthesis | 18,23 | 0,119 |
| SYBR | 31,25ng Hela RNA | 5µl of cDNA synthesis | 19,01 | 0,630 | 5µl of 1:2 dilution from Template above equates to 31,25ng Hela RNA in cDNA synthesis | 19,18 | 2,460 |
| SYBR | 1µg Hela RNA without Adapter Nucleic Acid | 5µl of cDNA synthesis | 33,85 | 3,802 | | | |

### Conclusion:

Quantifying of RNA amounts from about 30 ng up to 1µg is possible. This represents the typical range of RNA amounts used for cDNA synthesis.

### Example 2: Proof of accurate titration of adapter RNA or DNA with Uracial bases in probe based Realtime PCR. Confirmation of correlation between Ct-Value and dilution factor.

In this experiment the accurate titration of the adapter RNA is demonstrated. Furthermore the correlation between Ct-value and dilution factor is shown, demonstrating high PCR efficiency. For this purpose the components from table 12 were mixed as shown in table 13. Subsequently the protocol of table 14 was performed. PCR was performed on a Applied Biosystems 7500 Realtime PCR System. After this the run data were analyzed with the appropriate software. The results are shown in table 15 and figure 5.

| Table 12: Components and material numbers for probebased Realtime PCR setup | |
|---|---|
| QuantiTect Virus NR Mastermix, 5x | Components of QIAGEN - QuantiTect Virus + ROX Vial Kit Material No. 211033 |
| ROX Dye Solution, 50x | |
| RNAse free water | |
| HUM-Uni | AAC GAG ACG ACG ACA GAC |
| HBSR 1 | GGT GAG TGA TTG GAG GGT TG |
| Adapter Probe | AGC ACA TCA GAG CCC TGC GAT GA |

| Table 13: Setup for probe based Realtime PCR | |
|---|---|
| Components | Final concentration |
| QuantiTect Virus NR Mastermix. 5x | 1x |
| HUM-Uni | 0.4µM |
| HBSR 1 | 0.4µM |
| Adapter Probe | 0.2µM |
| ROX Dye Solution. 50x | 1x |
| QT RT Mix. 100x | 1x |
| RNAse free water | Top up to 20µl per reaction |
| Final reaction volume | 25µl |

| Table 14: Protocol for probe based Real-time PCR | | |
|---|---|---|
| Reverse transcription | 50°C for 20 minutes | |
| PCR initial reactivation | 95°C for 5 minutes | |
| Denaturing | 95°C for 15 seconds | 40x |
| Annealing/Extension | 60°C for 45 seconds | |

| Table 15: Results of probe based Real-time PCR | | | | |
|---|---|---|---|---|
| Detector | Template | Ct | Mean Ct | CV in % |
| FAM-BHQ | 6000000000 | 10.06 | 9.99 | 1.062 |
| | | 9.91 | | |
| FAM-BHQ | 600000000 | 13.6 | 13.57 | 0.365 |
| | | 13.53 | | |
| FAM-BHQ | 60000000 | 17.36 | 17.38 | 0.122 |
| | | 17.39 | | |
| FAM-BHQ | 6000000 | 21.29 | 21.30 | 0.066 |
| | | 21.31 | | |
| FAM-BHQ | 600000 | 25.28 | 25.28 | 0.000 |
| | | 25.28 | | |
| FAM-BHQ | 60000 | 28.93 | 28.94 | 0.049 |
| | | 28.95 | | |
| FAM-BHQ | 6000 | 32.82 | 32.72 | 0.454 |
| | | 32.61 | | |
| FAM-BHQ | 600 | 36.26 | 36.66 | 1.543 |
| | | 37.06 | | |

### Example 3: Analysis of cDNA generated in Example 1 via probe based Real-time PCR to demonstrate the correlation between Ct-Value and the amount of RNA used in cDNA synthesis

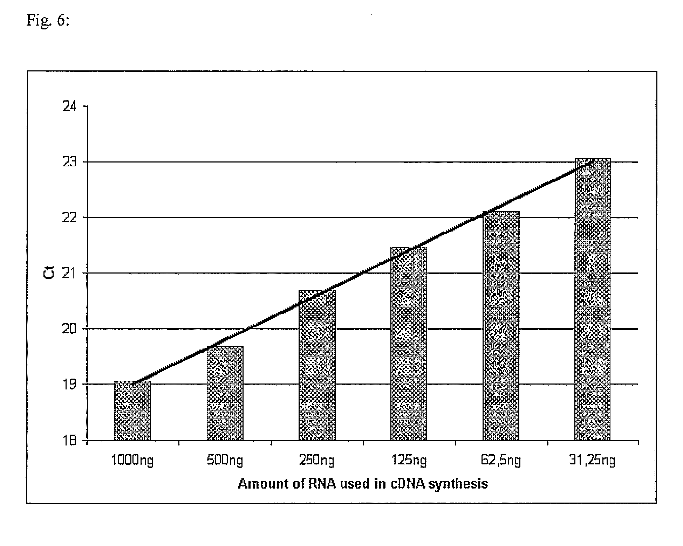

After inactivation of the enzyme (see example 1 table 2: 5 minutes at 95°C) an aliquot of the cDNA synthesis reaction was applied in the following Real-time PCR. The detection in Real-time PCR was carried out with probe based chemistry. Table 17 shows the setup for a probe based Real-time PCR reaction. The PCR reaction was prepared with the components from table 16. Afterwards the cycling shown in table 18 was performed. Real-time PCR was performed on a Applied Biosystems 7500 Real-time PCR System. Subsequently the run data were analyzed with the appropriate instrument software. The results are shown in table 19, 20 and in figure 6.

| Table 16:Components and material numbers for probe-based Real-time PCR setup | |
|---|---|
| QuantiTect Virus NR Mastermix, 5x | Components of QIAGEN - QuantiTect Virus + ROX Vial Kit Material No. 211033 |
| ROX Dye Solution, 50x | |
| RNAse free water | |
| HUM-Uni | AAC GAG ACG ACG ACA GAC |
| HBSR 1 | GGT GAG TGA TTG GAG GGT TG |
| Adapter Probe | AGC ACA TCA GAG CCC TGC GAT GA |

| Table 17: Setup for probe based Real-time PCR | |
|---|---|
| Components | Final concentration |
| QuantiTect Virus NR Mastermix, 5x | 1x |
| HUM-Uni | 0.4µM |
| HBSR 1 | 0.4µM |
| Adapter Probe | 0.2µM |
| ROX Dye Solution, 50x | 1x |
| RNAse free water | Top up to 20µl per reaction |
| Final reaction volume | 25µl |

| Table 18: Protocol for probe based Real-time PCR | | |
|---|---|---|
| PCR initial reactivation | 95°C for 5 minutes | |
| Denaturing | 95°C for 15 seconds | 40x |
| Annealing / Extension | 60°C for 45 seconds | |

| Table 19: Results of probe based Real-time PCR from a 500µl cDNA synthesis reaction | | | | | |
|---|---|---|---|---|---|
| Detector | Amount of RNA used in cDNA synthesis | Template in Real-Time PCR | Ct | Mean Ct | CV in % |
| FAM- | 1µg Hela RNA | 5µl of cDNA | 19.01 | 19.05 | 0.297 |
| BHQ | | synthesis | 19.09 | | |
| FAM- | 500ng Hela | 5µl of cDNA | 19.49 | 19.69 | 1.436 |
| BHQ | RNA | synthesis | 19.89 | | |
| FAM- | 250ng Hela | 5µl of cDNA | 20.76 | 20.68 | 0.547 |
| BHQ | RNA | synthesis | 20.60 | | |
| FAM- | 125ng Hela | 5µl of cDNA | 21.57 | 21.47 | 0.692 |
| BHQ | RNA | synthesis | 21.36 | | |
| FAM- | 62.5ng Hela | 5µl of cDNA | 22.09 | 22.12 | 0.192 |
| BHQ | RNA | synthesis | 22.15 | | |
| FAM- | 31.25ng Hela | 5µl of cDNA | 23.07 | 23.06 | 0.092 |
| BHQ | RNA | synthesis | 23.04 | | |
| FAM- | 1µg Hela RNA | 5µl of cDNA | 35.73 | 35.91 | 0.709 |
| BHQ | without Adapter Nucleic Acid | synthesis | 36.09 | | |

| Table 20: Results of probe based Real-time PCR from a 2µl cDNA synthesis reaction | | | | | |
|---|---|---|---|---|---|
| Detector | Amount of RNA used in cDNA synthesis | Template in Real-Time PCR | Ct | Mean Ct | CV in % |
| FAM-BHQ | 40ng Hela RNA | 5µl of 1:10 diluted cDNA synthesis reaction | 18.90 | 18.93 | 0.187 |
| | | | 18.95 | | |
| FAM-BHQ | 8ng Hela RNA | 5µl of 1:10 diluted cDNA synthesis reaction | 21.22 | 21.17 | 0.368 |
| | | | 21.1 | | |
| FAM-BHQ | 1µg Hela RNA ohne Adapter Nukleinsäure | 5µl of 1:10 diluted cDNA synthesis reaction | Undeter mined Undeter mined | - | - |

### Conclusion:

Quantifying of RNA amounts from 8 ng up to 1 µg is possible. This represents the typical range of RNA amounts used for cDNA synthesis.

### Description of drawings

The appended figures describe illustrative embodiments of the invention and are not limiting the scope of the invention. In these embodiments, the following definitions apply:
mRNA: may be poly-adenylated messenger RNA as naturally occurring from different species. This can also be any RNA sample, naturally with our without poly-A tail, wherein a poly-nucleotide tail is added in vitro by a suitable method, e.g. an enzyme, preferably a poly-A-polymerase.
1-x describes the lengths of the poly-nucleotide-tail, as naturally occurring or added in vitro. Suitable length varies from 1 to several thousand nucleotides.
A[A]AAAA describes the poly-nucleotide-tail, which can the naturally occurring poly-A-tail, or a poly-nucleotide tail added in vitro by a suitable method. 3' OH is available to allow enzymatic extension.

The adapter nucleic acid is a nucleic acid oligonucleotide, RNA or DNA or an analogous form thereof. It comprises an element hybridizing to the poly-nucleotide-tail, which can the naturally occurring poly-A-tail, under the given reaction conditions. In case the mRNA carries a poly-A-Tail, the hybridizing element preferably comprises bases complementary to the tail, i.e. in the case of a poly-A sequence, U and/or T bases. In a preferred embodiment, the lengths of the hybridizing element is between 1 and 150, more preferably between 2 and 100, most preferably between 4 and 80 nucleotides. Useful lengths may be limited by available production or synthesis technologies.

The adapter nucleic acid in addition comprises an adapter sequence. The adapter sequence is preferably selected to have sufficient lengths to allow for specific detection of the cDNA in a detection method, generated using this sequence as template. One preferred detection method is PCR. In this case, the adapter sequence has a sufficient length allowing for at least one PCR primer to hybridize, more preferably two PCR primers and optionally one or more sequence specific probes. The 3' OH group of this adapter nucleic acid can be blocked to prevent extension or can be free, to allow cDNA synthesis. Appropriate methods to block the 3' OH are standard methods and known in the art. Suitable methods may be 3' phosphate group, inverse base, aliphatic linker, amino modifier, or others.

The enzyme is at least one enzyme allowing cDNA synthesis using the poly-nucleotide tail as primer and the adapter nucleic acid as template. Different enzymes are possible dependent on the type of nucleic acids used in the poly-A-Tail or the adapter nucleic. It may originate from mesophilic or thermophilic organisms, providing activity in a temperature range from 5°C to 100°C, more preferably from 10°C-80°C, most preferably from 15°C to 75°C. dNTI's are desoxynucleotidetriphosphates required for DNA synthesis. The mixture may optionally contain modified nucleotides or labeled nucleotides or nucleotide analoga.

Buffer is a suitable buffer solution providing one or more buffer substances, a suitable pH, cations, cofactors like Mg²⁺, providing reaction conditions for the cDNA synthesis at the given reaction temperature and the given one or more enzymes.

Figure 1: Schematic illustration of the method for normalized quantification of RNA using an adapter nucleic acid.

The method of figure 1 may be used for the normalization of the quantity of an RNA to be quantified in a sample to
(a) the total quantity of RNA in the sample; or to
(b) the total quantity of a specific class of RNA in the sample,
comprising the steps of
(i) providing a sample containing an RNA to be quantified (see A; referred to as mRNA);
(ii) adding a first nucleic acid probe (referred to as adapter nucleic acid in (A)), to the sample under conditions allowing for hybridization of at least a terminal region of said first nucleic acid probe (poly-U-part of adapter nucleic acid in (B)) to specific binding sites of RNA (poly-A-tail of mRNA in (B)) in the sample such that double-stranded nucleic acids are created, wherein said first nucleic acid probe (referred to as adapter nucleic acid in (A) and (B)) comprises one or more primer binding sites and a probe binding site;
(iii) reverse transcribing the RNA in the sample, wherein the terminal region of the RNA serves as primer for cDNA synthesis and said first probe serves as template (suitable enzyme, desoxynucleotidtriphosphate and buffer, optional other primers and ingredients, added at step (A), cDNA synthesis reaction described in (B));
(iv) quantifying the total amount of RNA in the sample or the total amount of the specific class of RNA in the sample using a second probe substantially complementary to a region of the DNA transcribed from said RNA (referred to as adapter cDNA in (C));
(v) optionally quantifying the RNA to be quantified using a third probe which is substantially complementary to a defined region of the RNA to be quantified; and
(vi) normalizing the quantity of the RNA to be quantified by determining the ratio of the quantity of the RNA to be quantified to the total quantity of RNA in the sample or the total quantity of the specific class of RNA in the sample.

The method is suitable for detection of the cDNA generated from the adapter nucleic acid in subsequent reactions, like e.g. polymerase chain reaction (PCR or real-time PCR) using an intercalating fluorescent dye like for example SybrGreen or a sequence specific probe binding on one of the strands, like for example a Taqman probe to be used in a 5'-3' exonuclease assays.

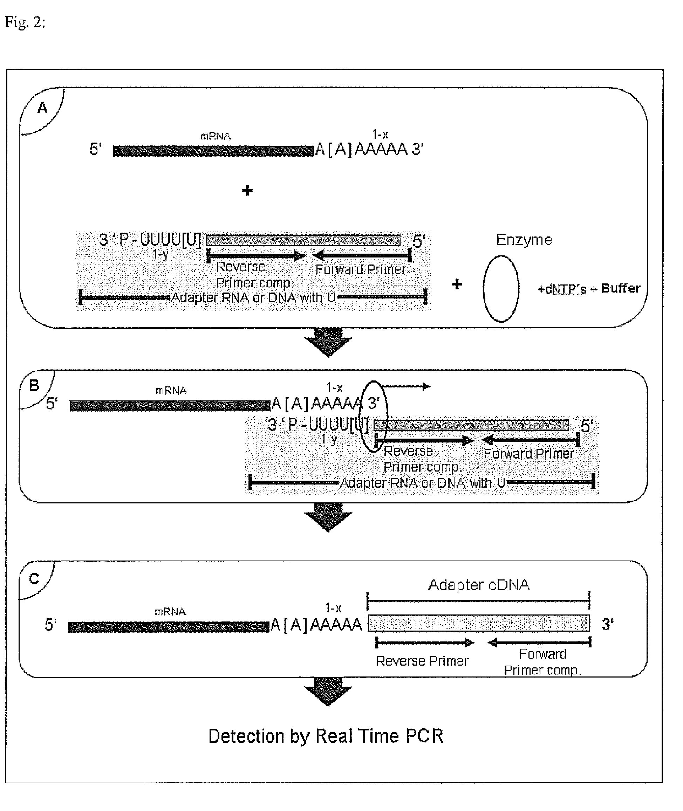

Figure 2: Schematic illustration of a preferred embodiment of the method for normalized quantification of RNA using an adapter nucleic acid and detection by amplification, preferably PCR, more preferably real-time PCR, using an intercalating fluorescent dye. A specific embodiment of this methodology is shown in example 1.

The method of figure 2 may be used for the normalization of the quantity of an RNA to be quantified in a sample to
(a) the total quantity of RNA in the sample; or to
(b) the total quantity of a specific class of RNA in the sample,
comprising the steps of
(i) providing a sample containing an RNA to be quantified (see A; referred to as mRNA);
(ii) adding a first nucleic acid probe (referred to as adapter nucleic acid in (A)), to the sample under conditions allowing for hybridization of at least a terminal region of said first nucleic acid probe (poly-U-part of adapter nucleic acid in (B)) to specific binding sites of RNA (poly-A-tail of mRNA in (B)) in the sample such that double-stranded nucleic acids are created, wherein said first nucleic acid probe (referred to as adapter nucleic acid in (A) and (B)) comprises one or more primer binding sites and a probe binding site;
(iii) reverse transcribing the RNA in the sample, wherein the terminal region of the RNA serves as primer for cDNA synthesis and said first probe serves as template (suitable enzyme, desoxynucleotidtriphosphate and buffer, optional other primers and ingredients, added at step (A), cDNA synthesis reaction described in (B));
(iv) quantifying the total amount of RNA in the sample or the total amount of the specific class of RNA in the sample using a second probe substantially complementary to a region of the DNA transcribed from said RNA (referred to as adapter cDNA in (C));
(v) optionally quantifying the RNA to be quantified using a third probe which is substantially complementary to a defined region of the RNA to be quantified; and
(vi) normalizing the quantity of the RNA to be quantified by determining the ratio of the quantity of the RNA to be quantified to the total quantity of RNA in the sample or the total quantity of the specific class of RNA in the sample.

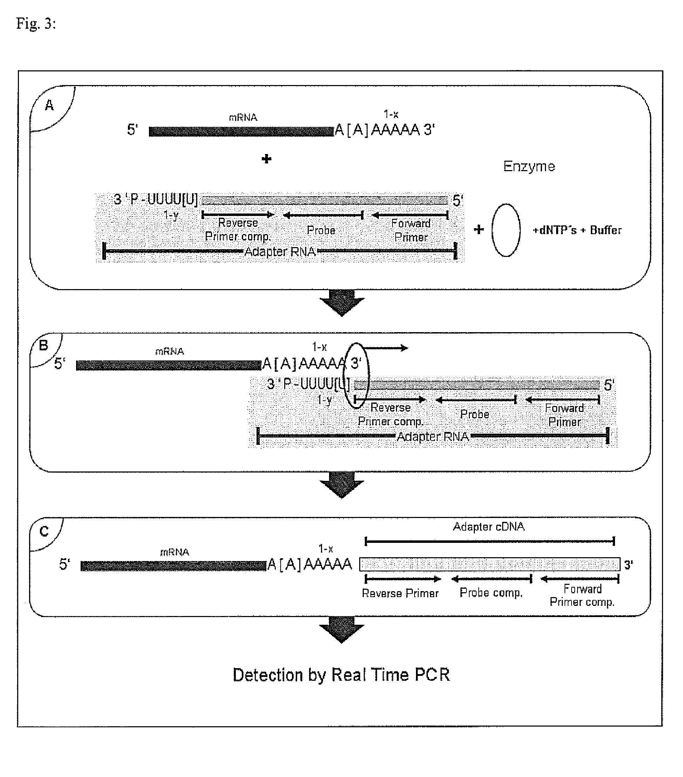

Figure 3: Schematic illustration of a preferred embodiment of the method for normalize quantification of RNA using adapter nucleic acid and detection by amplification, preferably PCR, more preferably real-time PCR using one or more sequence specific probes. A specific embodiment of this methodology is shown in example 5.

The method of figure 3 is may be used for the normalization of the quantity of an RNA to be quantified in a sample to
(a) the total quantity of RNA in the sample; or to
(b) the total quantity of a specific class of RNA in the sample,
comprising the steps of
(i) providing a sample containing an RNA to be quantified (see A; referred to as mRNA);
(ii) adding a first nucleic acid probe (referred to as adapter nucleic acid in (A)), to the sample under conditions allowing for hybridization of at least a terminal region of said first nucleic acid probe (poly-U-part of adapter nucleic acid in (B)) to specific binding sites of RNA (poly-A-tail of RNA in (B)) in the sample such that double-stranded nucleic acids are created, wherein said first nucleic acid probe (referred to as adapter nucleic acid in (A) and (B)) comprises one or more primer binding sites and a probe binding site;
(iii) reverse transcribing the RNA in the sample, wherein the terminal region of the RNA serves as primer for cDNA synthesis and said first probe serves as template (suitable enzyme, desoxynucleotidtriphosphate and buffer, optional other primers and ingredients, added at step (A), cDNA synthesis reaction described in (B));
(iv) quantifying the total amount of RNA in the sample or the total amount of the specific class of RNA in the sample using a second probe substantially complementary to a region of the DNA transcribed from said RNA (referred to as adapter cDNA in (C));
(v) optionally quantifying the RNA to be quantified using a third probe which is substantially complementary to a defined region of the RNA to be quantified; and
(vi) normalizing the quantity of the RNA to be quantified by determining the ratio of the quantity of the RNA to be quantified to the total quantity of RNA in the sample or the total quantity of the specific class of RNA in the sample.

Fig 4 shows the results obtained from the experiments described in example 1 (500 µl cDNA synthesis reaction, Detection by Real-time PCR with SYBR Green). Y-axis shows Ct values and x-axis the amounts of input RNA used in the respective cDNA synthesis reaction. The bars represent the mean values of the Ct values obtained in real-time PCR shown in table 11. 5µl of each of such cDNA synthesis reactions with the given amounts of input RNA were used as template in SybrGreen based real-time PCR (based bars), resulting in a template amount in each real-time PCR reaction being an RNA equivalent of 1/200 of the amounts given on the x-axis, As control, 5 µl a cDNA reaction with 1000 ng input RNA and a serial 1:2 dilution down to 31,25 ng were used a template (dotted grey bars), in order to demonstrate almost perfect linearity of Ct value and amount of template input. Linear regression lines for both conditions are shown.

Fig. 5 shows the results obtained from the experiments described in example 2, demonstrating linearity of Ct value and absolute number of adapter RNA molecules and PCR efficiency in the range of 100%. Y-axis shows Ct values and x-axis the number of input RNA molecules. The bars represent the mean values of the Ct values obtained in real-time PCR shown in table 15. Linear regression lines for both conditions are shown.

Fig. 6 shows the results obtained from the experiments described in example 3 (500µl cDNA synthesis, probe based Real-time PCR). Y-axis shows Ct values and x-axis the amounts of input RNA used in the respective cDNA synthesis reaction. The bars represent the mean values of the Ct values obtained in real-time PCR shown in table 19. 5µl of each of such cDNA synthesis reactions with the given amounts of input RNA were used as template in Probe based real-time PCR, resulting in a template amount in each real-time PCR reaction being an RNA equivalent of 1/200 of the amounts given on the x-axis. Almost perfect linearity of Ct value and amount of template input could be demonstrated. Linear regression line is shown.

## Claims

1. A method for the normalization of the quantity of an RNA to be quantified in a sample to
(a) the total quantity of RNA in the sample; or to
(b) the total quantity of a specific class of RNA in the sample,
comprising the steps of
(i) providing a sample containing an RNA to be quantified;
(ii) adding a first nucleic acid probe to the sample under conditions allowing for hybridization of at least a terminal region of said first nucleic acid probe to specific binding sites of RNA in the sample such that double-stranded nucleic acids are created, wherein said first nucleic acid probe comprises one or more primer binding sites and optionally a probe binding site;
(iii) reverse transcribing the RNA in the sample, wherein the terminal region of the RNA serves as primer for cDNA synthesis and said first probe serves as template;
(iv) quantifying the total amount of RNA in the samples or the total amount of the specific class of RNA in the sample optionally using a second probe substantially complementary to a region of the DNA transcribed from said RNA;
(v) quantifying the RNA to be quantified optionally using a third probe which is substantially complementary to a defined region of the RNA to be quantified; and
(vi) normalizing the quantity of the RNA to be quantified by determining the ratio of the quantity of the RNA to be quantified to the total quantity of RNA in the sample or the total quantity of the specific class of RNA in the sample.

2. The method according to claim 1, wherein the RNA to be quantified is RNA selected from the group consisting of mRNA., rRNA, tRNA, nRNA, siRNA, snRNA, snoRNA, scaRNA, microRNA, dsRNA, ribozyme, riboswitch and viral RNA and wherein the specific class of RNA is selected from the group consisting of mRNA, rRNA, tRNA, nRNA, siRNA, snRNA, snoRNA, scaRNA, microRNA, dsRNA, ribozyme, riboswitch and viral RNA.

3. The method according to claim 2, wherein the RNA to be quantified is RNA and the specific class of RNA is mRNA.

4. The method according to claim 3, wherein the specific binding site is a poly-A sequence or a poly-A tail.

5. The method according to any one of the claims 1 to 4, wherein an RNase is added to the sample after the reverse transcription step under conditions allowing for digestion of RNA.

6. The method according to claim 5, where the RNase is selected from the group consisting of RNase A, RNase H, RNase I, RNase T, and RNase activity of reverse transcriptase.

7. The method according to any one of the claims 1 to 6, wherein said first nucleic acid probe is a DNA in which dT nucleotides are replaced by dU nucleotides and wherein a step of incubating the samples with uracil DNA glycosylase under conditions allowing for hydrolysis of the N-glycosylic bond between the uracil and sugar, is performed before quantifying the total amount of RNA or the total amount of a specific class of RNA.

8. The method according to any one of the claims 1 to 7, wherein the 3' end of said first nucleic acid probe is blocked.

9. The method according to any one of the claims 1 to 8, wherein quantification comprises quantitative real-time PCR.

10. The method according to claim 9, wherein the quantification comprises quantitative real-time reverse transcription PCR.

11. The method according to any one of the claims 9 or 10, wherein reverse transcribing and quantifying are performed in the same reaction container.

12. The method according to claim 11, wherein the reverse transcriptase is a polymerase also used for amplification during the quantification steps.

13. The method according to any one of the claims 1 to 12, wherein selective primers or random primers are used in quantitative real-time PCR.

14. The method according to any one of the claims 1 to 13, wherein the second and third nucleic acid probe are labelled with one or more fluorescent dye(s) and wherein the quantifying steps comprise detecting fluorescence signals in the sample.

15. The method according to any one of the claims 1 to 14 for the normalization of gene expression levels.

16. The method according to any one of the claims 1 to 15, wherein additionally a pre-quantified RNA is added to the sample and wherein the quantity of said pre-quantified RNA is determined in the quantifying steps.

17. The method according to claim 16, wherein the pre-quantified RNA is a mRNA.

18. A kit for performing the method of any one of the claims 1 to 17, wherein the kit comprises:
(i) a first nucleic acid probe substantially complementary to defined regions of RNA or a specific class of RNA in the sample, wherein the first nucleic acid probe comprises one or more primer binding sites and a probe binding site; and
(ii) a second nucleic acid probe substantially complementary to said probe binding site on said first nucleic acid probe.

19. The use of the methods according to any one of the claims 1 to 17 or the kit according to claim 18 for the normalization of the quantity of a specific nucleic acid to the quantity of a reference nucleic acid.

20. The use of the methods according to any one of the claims 1 to 17 or the kit according to claim 18 for gene expression analysis.
